# Europäisches Patentamt
## European Patent Office
## Office européen des brevets

(19)

(11) Publication number: **0 011 032**
**B1**

(12) **EUROPEAN PATENT SPECIFICATION**

(45) Date of publication of patent specification: **23.06.82**

(51) Int. Cl.³: **A 61 K 39/29, C 07 G 7/00**

(21) Application number: **79400800.3**

(22) Date of filing: **26.10.79**

(54) Method for isolation of HBsAg.

(30) Priority: **30.10.78 US 955863**

(43) Date of publication of application:
**14.05.80 Bulletin 80/10**

(45) Publication of the grant of the patent:
**23.06.82 Bulletin 82/25**

(84) Designated Contracting States:
**AT BE CH DE FR GB IT LU NL SE**

(56) References cited:
**GB - A - 1 442 564**
**US - A - 4 029 764**
**US - A - 4 031 203**

**Chemical Abstracts vol. 88, no. 25 19 June 1978 Columbus, Ohio, USA Y. KAMIMURA et al. "Specific antibody for hepatitis B surface antigen (HBsAg)" page 544, column 1, abstract no. 187992g**

(73) Proprietor: **MERCK & CO. INC.**
**126, East Lincoln Avenue P.O. Box 2000**
**Rahway, New Jersey 07065 (US)**

(72) Inventor: **McAleer, William J.**
**717 Marietta Drive**
**Ambler, Pennsylvania 19002 (US)**
Inventor: **Wasmuth, Edward H.**
**25 Church Avenue**
**Telford, Pennsylvania 18969 (US)**

(74) Representative: **Corre, Jacques Denis Paul et al,**
**Cabinet Regimbeau 26, Avenue Kléber**
**F-75116 Paris (FR)**

Courier Press, Leamington Spa, England.

Method for isolation of HB$_s$Ag

The present invention is concerned with a method for isolating HB$_s$Ag.

According to the present invention, high purity HB$_s$Ag is injected into guinea pigs to stimulate formation of HB$_s$Ab-positive serum. Gamma globulins containing HB$_s$Ab are separated from the serum and the globulin fraction containing HB$_s$Ab is isolated and coupled to a linear polysaccharide in the form of a fixed bed, e.g. a Sepharose® gel. HB$_s$Ag from HB$_s$Ag-positive plasma is adsorbed on the gel by formation of an HB$_s$Ag-HB$_s$Ab complex. HB$_s$Ag is eluted from the column and dialysed.

More particularly, the present method for isolating HB$_s$Ag comprises recovering the serum from a susceptible animal species which has been injected with highly purified HB$_s$Ag having about the following properties:

| | |
|---|---|
| $E_{1\%}$ | 52.3 |
| $OD_{250\ nm}$ | 0.115 |
| $OD_{260\ nm}$ | 0.082 |
| Lowry protein ($\mu g/ml$) | 22.0 |
| CF units/ml | 128 |
| RIA units/ml | 8,000 |
| RIA units per $\mu g$ protein | 364 |
| Particle diameter | 18—22 nm |

to stimulate the formation of HB$_s$Ab-positive serum, treating the serum to separate a 7S globulin fraction containing HB$_s$Ab, further purifying the HB$_s$Ab in the fraction by chromatographic separation, coupling the purified HB$_s$Ab to a linear polysachharide in the form of a fixed bed, contacting the HB$_s$Ab in the fixed bed with HB$_s$Ag-positive serum thereby forming an HB$_s$Ab-HB$_s$Ag immune complex, and separating HB$_s$Ag from the complex.

In the background art British patent 1,442,564 describes a process for preparing hepatitis B (surface) antigen vaccine by heating a solution of human plasma containing the antigen at 60—120°C in the presence of a stabilizer selected from a monsaccharide, disaccharide, sugar alcohol or mixture thereof, and removing the stabilizer. The present process does not involve heating a solution of HB$_s$Ag-containing plasma in the presence of such a stabilizer, nor is there any disclosure in this British patent of the feature of the present invention which involves contacting HB$_s$Ab which is coupled to a linear polysachharide in the form of a fixed bed with HB$_s$Ag-positive serum.

Furthermore Chemical Abstracts 88:187992g discloses that human plasma containing HB$_s$Ab was fractionated to give a crude immune globulin (Ig) which was adsorbed on CM-cellulose and subjected to gradient elution with phosphate buffer and NaCl to give an electrophoretically slow Ig fraction containing high antibody activity with low non-specific reactivity. There is no disclosure in this abstract of contacting HB$_s$Ab coupled to a linear polysaccharide in a fixed bed with HB$_s$Ag-positive serum.

According to the present invention highly purified HB$_s$Ag antigen is injected into a susceptible animal species. By a susceptible animal species is meant one which forms HB$_s$Ab in response to injection of HB$_s$Ag, e.g., guinea pigs, grivet monkeys and chimpanzees. The antigen may be present in a composition containing a physiologically acceptable medium, e.g. saline. An adjuvant, e.g. Freund's complete adjuvant, Adjuvant 65 (described in U.S. patent 3,149,036) or Adjuvant 65-4 (described in U.S. Patents 3,983,228 and 4,069,313), or alum may also be present. The animal is bled at from 12 to 16 weeks post inoculation to obtain HB$_s$Ab-positive serum.

By highly purified HB$_s$Ag is meant a spherical particle substantially equivalent to that described in U.S. patent 4,017,360 having about the following properties:

| | |
|---|---|
| $E_{1\%}$ | 52.3 |
| $OD_{250\ nm}$ | 0.115 |
| $OD_{260\ nm}$ | 0.082 |
| Lowry protein ($\mu g/ml$) | 22.0 |
| CF units/ml | 128 |
| RIA units/ml | 8,000 |
| RIA units per $\mu g$ protein | 364 |
| Particle diameter | 18—22 nm |

The serum is treated to separate a globulin fraction containing HB$_s$Ab, e.g. by dialysis against ammonium sulfate to precipitate gamma globulins containing HB$_s$Ab. Preferably the ammonium sulfate is from about 2M to about 4M. The dialysis is preferably carried out at lowered temperatures of from 10° down to above the freezing point of the solution. The ammonium sulfate is removed by a further dialysis treatment employing, e.g., phosphate buffered saline (PBS).

The dialyzed gamma globulins are then treated to isolate the 7S globulin fraction containing purified HB$_s$Ab. This treatment is effected conveniently by means of chromatographic separation, e.g. by means of a cross-linked dextran such as Sephadex® G-200, or a cellulose, such as DEAE-cellulose.

The isolated 7S globulin fraction which is recovered as eluate from the chromatographic separation is coupled to activated CNBr Sepharose® 4B in a suitable buffer and the complex is poured into a column and rinsed. An example of a suitable coupling buffer is sodium citrate having a molarity of from 0.1 to 2. The coupling buffer is removed, e.g., by rinsing with a neutral isotonic solution, e.g., tris buffer. The latter may also be used to rinse the column.

HB$_a$Ag-positive plasma which has been converted to serum is dialyzed against the same buffer used to remove the coupling buffer, and passed into the column to adsorb $HB_sAg$ by forming an $HB_sAb$-$HB_sAg$ complex. The column is flushed once to free un-adsorbed sample, e.g. with 0.1 M tris buffer, and again to remove non-specifically bound protein molecules, e.g. with 0.5 M tris buffer. The $HB_sAb$-$HB_sAg$ complex is then dissociated, e.g. by treatment with NaSCN, and dialyzed to yield purified $HB_sAg$.

The following examples illustrate the present invention without, however, limiting the same thereto.

Example 1

$HB_sAg$ prepared according to Example 1 of U.S. Patent 4,017,360 is adsorbed on alum. Doses, 1.0 ml each, containing 20 $\mu g/ml$ are injected into guinea pigs at 0, 14 and 56 day intervals. The animals are bled at the 12th and 16th week following first injection to obtain $HB_sAb$-positive serum 70 ml of which is dialyzed against 3M ammonium sulfate overnight at 5°C to precipitate gamma globulin containing $HB_sAb$. The precipitated globulin is dissolved in phosphate buffered saline (PBS) to a volume of 15 ml and dialyzed against PBS to remove ammonium sulfate. The dialyzed globulin is then passed through a 5×90 cm Sephadex® G-200 column, equilibrated with PBS, to isolate the 7S globulin fraction. The 7S globulin fraction is dialyzed overnight against 3.0 l of 0.1 M citrate buffer, pH 6.5. The activated CNBr Sepharose® 4B is prepared with a final rinse in 2.0 l of 0.1 M citrate buffer, pH 6.5. The 7S globulin fraction and the activated Sepharose® 4B are reacted together at 5°C for 20 hours with gentle mixing. The Sepharose 4B-$HB_sAg$ complex is collected on a glass filter and rinsed with 1.0 l of 0.1 M Tris saline buffer, pH 7.4. The Sepharose® 4B-$HB_sAb$ complex is poured into a 1.6×70 cm column and rinsed again with 100 ml of 0.1 M Tris buffer, pH 7.4. $HB_sAg$-positive plasma, 15 ml, which has been converted to serum and dialysed against 0.1 M Tris saline buffer, pH 7.4, is passed into the column to adsorb $HB_sAg$ by formation of a $HB_sAb$-$HB_sAg$ complex. The complex is washed sequentially with 50 ml of 0.1 M and 50 ml 0.5 M Tris saline buffer, pH 7.4, and eluted with 50 ml 3M NaSCN, pH 7.4. The $HB_sAg$ containing fractions are pooled and dialysed against 3 l of 0.1 M Tris saline buffer, pH 7.4.

Claim

A method for isolating $HB_sAg$ comprising recovering the serum from a susceptible animal species which has been injected with highly purified $HB_sAg$ having about the following properties:

| | |
|---|---|
| $E_{1\%}$ | 52.3 |
| $OD_{250\ nm}$ | 0.115 |
| $OD_{260\ nm}$ | 0.082 |
| Lowry protein ($\mu g/ml$) | 22.0 |
| CF units/ml | 128 |
| RIA units/ml | 8,000 |
| RIA units per $\mu g$ protein | 364 |
| Particle diameter | 18—22 nm |

to stimulate the formation of $HB_sAb$-positive serum, treating the serum to separate a 7S globulin fraction containing $HB_sAb$, further purifying the $HB_sAb$ in the fraction by chromatographic separation, coupling the purified $HB_sAb$ to a linear polysaccharide in the form of a fixed bed, contacting the $HB_sAb$ in the fixed bed with $HB_sAg$-positive serum thereby forming an $HB_sAb$-$HB_sAg$ immune complex, and separating $HB_sAg$ from the complex.

Patentanspruch

Verfahren zum Isolieren von $HB_sAg$ durch Gewinnen des Serums aus einer empfindlichen Tierspecies der hochgereinigtes $HB_sAg$ mit etwa folgenden Eigenschaften

| | |
|---|---|
| $E_{1\%}$ | 52,3 |
| $OD_{250\ nm}$ | 0,115 |
| $OD_{260\ nm}$ | 0,082 |
| Lowry-Protein ($\mu g/ml$) | 22,0 |
| CF-Einheiten/ml | 128 |
| RIA-Einheiten/ml | 8000 |
| RIA-Einheiten pro $\mu g$ Protein | 364 |
| Teilchendurchmesser | 18—22 nm |

injiziert worden war zur Stimulierung der Bildung von $HB_sAk$-positivem Serum, Behandeln des Serums zur Abtrennung einer 7S-Globulinfraktion, enthaltend $HB_sAk$, weitere Reinigung des $HB_sAk$ in der Fraktion durch chromatographische Trennung, Kuppeln des gereinigten $HB_sAk$ an ein lineares Polysacharid in Form eines Festbetts, In-Kontakt-Bringen des $HB_sAk$ in dem Festbett mit $HB_sAg$-positivem Serum, um dadurch einen $HB_sAk$-$HB_sAg$-Immun-Komplex zu bilden, und Abtrennung des $AB_sAg$ von dem Komplex.

Revendication

Procédé pour isoler $HB_sAg$ impliquant de recueillir le sérum à partir d'une expèce animale sensible à laquelle on a injecté du $HB_sAg$ haute-

ment purifié ayant approximativement les propriétés suivantes:

| | |
|---|---|
| $E_{1\%}$ | 52,3 |
| $OD_{250\ nm}$ | 0,115 |
| $OD_{260\ nm}$ | 0,082 |
| Protéine de Lowry ($\mu$g/ml) | 22,0 |
| Unités CF/ml | 128 |
| Unités RIA/ml | 8000 |
| Unités RIA/$\mu$g de protéine | 364 |
| Diamètre particulaire | 18—22 nm |

pour stimuler la formation de sérum $HB_sAb$-positif, de traiter le sérum pour séparer une fraction globulinique 7S contenant $HB_sAb$, puis de purifier le $HB_sAb$ dans la fraction par séparation chromatographique, de coupler le $HB_sAb$ purifié à un polysaccharide linéaire sous la forme d'un lit fixé, de mettre en contact le $HB_sAb$ dans le lit fixé avec du sérum $HB_sAg$-positif, formant ainsi un complexe immun $HB_sAb$-$HB_sAg$, et de séparer le $HB_sAg$ du complexe.